# EUROPÄISCHE PATENTSCHRIFT

(11) **EP 0 548 737 B1**
(45) Veröffentlichungstag und Bekanntmachung des Hinweises auf die Patenterteilung: **27.03.1996**
(21) Anmeldenummer: 92121285.8
(22) Anmeldetag: 15.12.1992
(51) Int. Cl.: C07C 59/60, C07C 51/09

(54) **Verfahren zur Herstellung von Oxadimethacrylsäure**
Process for preparing oxadimethyl acrylic acid
Procédé de préparation d'acide oxadiméthyl acrylique

(30) Priorität: 24.12.1991 DE 4142913
(43) Veröffentlichungstag der Anmeldung: 30.06.1993
(73) Patentinhaber: BASF Aktiengesellschaft, D-67063 Ludwigshafen (DE)
(72) Erfinder: Besecke, Siegmund, Dr., W-3250 Hameln (DE); Lauke, Harald, Dr., W-6800 Mannheim 1 (DE); Deckers, Andreas, Dr., W-6700 Ludwigshafen (DE)

(56) Entgegenhaltungen:
- US-A- 4 889 948

## Beschreibung

Die Erfindung betrifft ein verbessertes Verfahren zur Herstellung von 2,2'-[Oxybis(methylen)]bis-2-propensäure

CH₂=C(COOH)CH₂-O-CH₂C(COOH)=CH₂

mit einer Reinheit von mindestens 99 %, durch Hydrolyse der entsprechenden Ester in wäßrigem Milieu und anschließendes Ansäuern des entstandenen Salzes.

Oxadimethacrylverbindungen der allgemeinen Formel I

CH₂=C(COOZ)CH₂-O-CH₂C(COOZ)=CH₂ I

in der Z = H, Me, Et, n-Butyl, i-Butyl, tert.-Butyl, Neopentyl, Benzyl, Phenethyl, Trimethylcyclohexyl und Tetrahydrofurfuryl bedeutet, sind aus der US-A 4,889,948, aus Polymer Preprints, American Chemical Society, Division of Polymer Chemistry 31(1) (1990) 503 und aus der dort zitierten Literatur bekannt.

Sie sind wegen ihrer Bifunktionalität als Monomerbausteine geschätzte Verbindungen, die vielfach Verwendung finden, beispielsweise als Monomere zur Herstellung von Homopolymeren, oder als Comonomere oder Vernetzer. Bislang stehen aber nur einige wenige in nicht ausreichender Menge zur Verfügung. Ein weiterer Nachteil besteht in der meist unzureichenden Reinheit dieser Verbindungen. So gibt es über die Stoffeigenschaften wie dem Schmelzpunkt der 2,2'-[Oxybis(methylen)]bis-2-propensäure unterschiedliche Angaben (s. US-A 4,889,948 und Polym.Lett., 25 (1987) 451).

2,2'-[Oxybis(methylen)]bis-2-propensäure ("Oxadimethacrylsäure") kann man nach Beispiel X in der US-A 4,889,948 durch basenkatalysierte Hydrolyse in einer wäßrigen Methanollösung und anschließendes Ansäuern des Salzes neben einer unbestimmten Menge an dem entsprechenden Homopolymeren der Oxadimethacrylsäure herstellen. Desweiteren führt die Polymerisation dieser verunreinigten Oxadimethacrylsäure zu gummiartigen, vernetzten, praktisch nicht weiterverarbeitbaren Polymeren.

Aufgabe der vorliegenden Erfindung war es daher, ein Verfahren zur Herstellung von reiner Oxadimethacrylsäure zur Verfügung zu stellen.

Demgemäß wurde ein verbessertes Verfahren zur Herstellung von 2,2'-[Oxybis(methylen)]bis-2-propensäure

CH₂=C(COOH)CH₂-O-CH₂C(COOH)=CH₂

mit einer Reinheit von mindestens 99 %, durch Hydrolyse der entsprechenden Ester in wäßrigem Milieu und anschließendes Ansäuern des entstandenen Salzes, gefunden, indem man die Hydrolyse in einer wäßrigen, basischen Lösung durchführt.

Nach den bisherigen Beobachtungen ist das gute Gelingen dieses Verfahrens von der Art der eingesetzten Oxadimethacrylsäureester der allgemeinen Formel I

CH₂=C(COOR¹)CH₂-O-CH₂C(COOR²)=CH₂ I

praktisch nicht abhängig, so daß die Esterreste R¹ und R² prinzipiell beliebig sein können, da sie ohnehin abgespalten werden. Als Substituenten kommen bevorzugt folgende Reste R¹ und R², die gleich oder verschieden sein können, mit der Maßgabe, daß sie nicht gleichzeitig Wasserstoff bedeuten, in Betracht:
Wasserstoff;
C₁-C₁₈-Alkyl, darunter vorzugsweise C₁-C₁₂-Alkyl wie Methyl, Ethyl, n- Propyl, i-Propyl, n-Butyl, sek.-Butyl, i-Butyl, tert.-Butyl, n-Pentyl, i- Pentyl, sek.-Pentyl, tert.-Pentyl, neo-Pentyl, n-Hexyl, n-Heptyl, n-Octyl, n-Nonyl, n-Decyl, n-Undecyl, n-Dodecyl und Stearyl, besonders bevorzugt C₁-C₄-Alkyl wie Methyl, Ethyl, n-Propyl, i-Propyl, n-Butyl, i-Butyl, sek.-Butyl, tert.-Butyl;
C₃-C₈-Cycloalkyl wie Cyclopropyl, Cyclopentyl, Cyclohexyl, Cycloheptyl, Cyclooctyl, 4-Methylcyclohexyl, 4-Methoxycyclohexyl, 2,4,6- Trimethylcyclohexyl;
C₃-C₈-Cycloalkyl-C₁-C₅-alkyl wie Cyclopropylmethyl, Cyclopentylmethyl, Cyclohexylmethyl, Cyclopropylethyl, Cyclopentylethyl, Cyclohexylethyl, Cyclopropylpropyl, Cyclopentylpropyl, Cyclohexylpropyl, Cyclopentylbutyl, Cyclohexylbutyl, Cyclopentylpentyl, Cyclohexylpentyl, Cyclooctylpentyl;
Hydroxy-C₁-C₅-alkyl wie Hydroxymethyl, 2-Hydroxyethyl, 2-Hydroxypropyl, 3-Hydroxypropyl, 4-Hydroxybutyl, 5-Hydroxypentyl, 2,2-Dimethyl-3-hydroxypropyl;
Amino-C₁-C₅-alkyl wie Aminomethyl, 2-Aminoethyl, 3-Aminopropyl, 4- Aminobutyl, 5-Aminopentyl;
N-C₁-C₄-Alkyl-amino-C₁-C₅-alkyl wie N-Methylaminomethyl, 2-(N-Methylamino)ethyl, 3-(N-Methylamino)propyl, 4-(N-Methylamino)butyl, 5-(N-Methylamino)pentyl, N-Ethylaminomethyl, N-n-Propylaminomethyl, N-n- Butylaminomethyl;
N,N-Di-(C₁-C₄-Alkyl)amino-C₁-C₅-alkyl wie N,N-Dimethylaminomethyl, 2-(N,N-Dimethylamino)ethyl, 3-(N,N-Dimethylamino) propyl, 4-(N,N-Dimethylamino)butyl, 5-(N,N-Dimethylamino)pentyl, N,N-Diethylaminomethyl, N,N-Di(n-Propyl)aminomethyl, N,N-Di(i-Propyl)aminomethyl, N,N-Di-(n-Butyl)aminomethyl, N-Ethyl-N- methyl-aminomethyl, N-Methyl-N-propylaminomethyl;
C₆-C₁₈-Aryl wie Phenyl, Naphthyl, Anthracenyl, Phenantrenyl, Azulenyl, Biphenylenyl, Triphenylenyl, bevorzugt Phenyl, wobei die Arylreste bis zu drei der unter R³ genannten Gruppen tragen können;
C₆-C₁₈-Aryl-C₁-C₄-alkyl, bevorzugt Phenyl-C₁-C₄-alkyl wie Benzyl, 2- Phenylethyl, 3-Phenylpropyl, 4-Phenylbutyl, besonders bevorzugt Benzyl, 2-Phenylethyl, 3-Phenylpropyl, wobei die Arylgruppen bis zu drei der unter R³ genannten Gruppen tragen können;
- R³: Halogen wie Fluor, Chlor, Brom und Iod, C₁-C₂₂-Alkyl wie Methyl, Ethyl, n-Propyl, i-Propyl, n-Butyl, sek.-Butyl, i-Butyl, tert.-Butyl, n-Pentyl, i-Pentyl, sek.-Pentyl, tert.-Pentyl, neo-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosyl, n-Heneicosyl und n-Docosyl, vorzugsweise C₁-C₁₂-Alkyl wie Methyl, Ethyl, n-Propyl, i-Propyl, n-Butyl, sek.-Butyl, i-Butyl, tert.-Butyl, n-Pentyl, i-Pentyl, sek.-Pentyl, tert.-Pentyl, neo-Pentyl, n-Hexyl, n-Heptyl, n-Octyl, n-Nonyl, n-Decyl, n-Undecyl, n-Dodecyl und Stearyl, besonders bevorzugt C₁-C₄-Alkyl wie Methyl, Ethyl, n-Propyl, i-Propyl, n-Butyl, i-Butyl, sek.-Butyl, tert.-Butyl; C₁-C₄-Alkoxy wie Methoxy, Ethoxy, n-Propoxy und n-Butoxy, Carboxy, C₁-C₄-Alkoxy-carbonyl wie Methoxycarbonyl, Ethoxycarbonyl, n-Propoxycarbonyl und n-Butoxycarbonyl, Aminocarbonyl, C₁-C₄-Alkylaminocarbonyl wie Methylaminocarbonyl, Ethylaminocarbonyl, n-Propylaminocarbonyl und n-Butylaminocarbonyl, Di-(C₁-C₄-Alkyl)aminocarbonyl wie Dimethylaminocarbonyl, Diethylaminocarbonyl, Di-(n-Propyl)aminocarbonyl und Di-(n-Butyl)aminocarbonyl, Nitrilo, Nitro, Amino, C₁-C₄-Alkylamino wie Methylamino, Ethylamino, n-Propylamino und n-Butylamino, Di-(C₁-C₄-alkyl)amino wie Dimethylamino, Diethylamino, Di-(n-Propyl)amino und Di-(n-Butyl)amino.

Besonders bevorzugte Verbindungen sind die Di-C₁-C₄-alkylester wie die Dimethyl-, Diethyl-, Dipropyl-, Dibutylester der Oxadimethacrylsäure, insbesondere der Dimethylester, 2,2'-[Oxybis(methylen)]bis-2-propensäure-dimethylester, als auch deren Mischester wie der Ethyl-methylester sowie Mischungen dieser Ester.

Nach dem erfindungsgemäßen Verfahren stellt man die Oxadimethacrylsäure dadurch her, daß man den Oxadimethacrylsäureester I, oder eine Mischung verschiedener dieser Ester, in basischer Lösung hydrolysiert und dann das entstandene Salz ansäuert. Danach kann man die ausgefallene Säure abtrennen und gegebenenfalls im sauren, wäßrigen Milieu umkristallisieren.

Als basische Lösung wählt man erfindungsgemäß wäßrige Lösungen von Alkalimetall- und Erdalkalimetallhydroxide wie Lithiumhydroxid, Natriumhydroxid, Kaliumhydroxid, Magnesiumhydroxid, Calciumhydroxid und Bariumhydroxid, bevorzugt Natrium- und Kaliumhydroxid, oder Ammoniak.

Als Polymerisationsinhibitoren verwendet man bevorzugt die üblichen wasserlösliche Verbindungen wie Hydrochinon, Hydrochinonmonoethylether und Cu(II)-Salze.

Zur Freisetzung der freien Säure setzt man dem Reaktionsmedium eine Säure, bevorzugt eine Mineralsäure wie Schwefelsäure, Salzsäure, Phosphorsäure und Salpetersäure, besonders bevorzugt Salzsäure, zu.

Das Molverhältnis von Base zu Ester wählt man im allgemeinen im Bereich von 1 bis 5, bevorzugt von 2 bis 4. Die Base setzt man in der Regel als wäßrige Lösung in Konzentrationen von 0,1 bis 40, vorzugsweise von 1 bis 20 Gew.-%, bezogen auf Wasser, ein.

Die Menge der Polymerisationsinhibitoren wählt man in der Regel im Bereich von 0 bis 0,1, vorzugsweise von 0 bis 0,05 Gew.-%, jeweils bezogen auf die Gesamtmenge der Reaktionsmischung.

Die Menge der zur Fällung eingesetzten Säure hängt von deren Stärke und Konzentration ab. In der Regel wählt man sie so, daß man das Salz enthaltende Medium, in der Regel das Reaktionsgemisch, auf einen pH-Wert im Bereich von 0,5 bis 2,0, bevorzugt von 0,5 bis 1,5, einstellt.

Die Wahl der Temperatur ist nach den bisherigen Beobachtungen nicht kritisch. In der Regel arbeitet man in einem Temperaturbereich von 10 bis 100°C unter einem Druck im Bereich von 70 bis 300 kPa. Man kann auch bei Temperaturen oberhalb von 100°C, in der Regel aber nicht höher als 200°C, in einem Druckreaktor hydrolysieren. Bevorzugt arbeitet man jedoch unter Atmosphärendruck in einem Bereich von 15 bis 50°C.

Die ausgefällte Oxadimethacrylsäure kann mit den üblichen Methoden wie Filtration, Dekantieren oder Zentrifugation abgetrennt und, falls erwünscht, gereinigt werden, beispielsweise indem man sie mit kaltem Wasser wäscht und anschließend trocknet. Nach den bisherigen Beobachtungen weist die so erhaltene Oxadimethacrylsäure eine Reinheit von mindestens 99 % auf.

Besonders reine Oxadimethacrylsäure, beispielsweise mit einem Gehalt an Nebenprodukten kleiner als 100 ppm, kann man vorzugsweise durch Umkristallisation erhalten. Hierzu löst man in der Regel die Oxadimethacrylsäure in 50 bis 100, bevorzugt 60 bis 100, besonders bevorzugt in 80 bis 100°C heißem Wasser und läßt sie anschließend bei Temperaturen im Bereich von 5 bis 30, vorzugsweise 10 bis 25°C, auskristallisieren. Man kann der Lösung Polymerisationsinhibitoren wie Hydrochinonmonomethylether in Mengen von 10 bis 20 ppm zusetzen. Des weiteren kann man die Lösung mit adsorbierenden Stoffen wie Aktivkohle, Kieselgur und Zeolithe versetzen, anschließend heiß filtrieren und dann zur Kristallisation abkühlen lassen.

Die Carbonsäuregruppen der Oxadimethacrylsäure kann man nach an sich bekannten Verfahren zu Ester-, Amid- und Keton-Gruppen weiterfunktionalisieren (s. Houben-Weyl, Methoden der organischen Chemie, Bd. VIII/III, Thieme, Berlin, 1952, S. 503 ff und S. 647 ff).

Des weiteren kann man Oxadimethacrylsäure nach an sich bekannten Methoden der Polymerisation (s. US-A 4,889,948) polymerisieren.

Nach dem erfindungsgemäßen Verfahren kann man 2,2'[Oxybis(methylen)]bis-2-propensäure in hochreiner Form herstellen. Oxadimethacrylsäure steht somit der gezielten Herstellung von Polymeren oder Copolymeren zur Verfügung. Ferner kann man aus ihr durch Umsetzung nach an sich üblichen Methoden, beispielsweise mit Alkoholen oder Säurechloriden, andere Oxadimethacrylate wie die entsprechenden Monoester oder gemischten Ester sowie andere Diester herstellen, die auf anderem Wege nicht oder in nur schlechten Ausbeuten zugänglich sind.

### Beispiel

### Darstellung von 2,2'[Oxybis(methylen)]bis-2-propensäure

Zu einer Lösung aus 15 g Natriumhydroxid in 135 g Wasser wurde eine Mischung aus 30 g (0,14 mol) 2,2'[Oxybis(methylen)]bis-2-propensäuredimethylester und 60 mg Hydrochinon gegeben und 19 h bei Raumtemperatur gerührt. Danach wurde mit konzentrierter Salzsäure ein pH-Wert von 1 eingestellt. Die dabei ausgefallene Säure wurde abfiltriert, dann in 160 g destilliertem Wasser bei 90°C gelöst, anschließend mit 10 g Aktivkohle versetzt und danach heiß filtriert. Beim Abkühlen auf Raumtemperatur fielen 24,5 g (94 %) hochreine 2,2'[Oxybis(methylen)]bis-2-propensäure mit einem Schmelzpunkt von 177°C an.
Gehalt an Nebenprodukten: <40 ppm, bestimmt durch GC/MS-Messungen.
- ¹H-NMR-Daten:: =CH₂ 5,6 und 5,95 ppm
-CH₂- 4,25 ppm

## Patentansprüche

1. Verfahren zur Herstellung von 2,2'-[Oxybis(methylen)]-bis-2-propensäure
CH₂=C(COOH)CH₂-O-CH₂C(COOH)=CH₂
mit einer Reinheit von mindestens 99 %, durch Hydrolyse der entsprechenden Ester in wäßrigem Milieu und anschließendes Ansäuern des entstandenen Salzes, dadurch gekennzeichnet, daß man die Hydrolyse in Wasser und in Gegenwart von Oxiden oder Hydroxiden von Alkali- oder Erdalkalimetallen oder Ammoniak durchführt.

2. Verfahren nach Anspruch 1, dadurch gekennzeichnet, daß man die Hydrolyse in Wasser und in Gegenwart von Natrium- oder Kaliumhydroxid durchführt.

3. Verfahren nach Anspruch 1 oder 2, dadurch gekennzeichnet, daß man einen Polymerisationsinhibitor mitverwendet.

4. Verfahren nach einem der Ansprüche 1 bis 3, dadurch gekennzeichnet, daß man als Ester einen oder mehrere 2,2'-[Oxybis(methylen)]bis-2-propensäure-di(C₁-C₄-alkyl)ester einsetzt.

5. Verfahren nach einem der Ansprüche 1 bis 4, dadurch gekennzeichnet, daß man das erhaltene Rohprodukt umkristallisiert.

## Claims

1. A process for preparing 2,2'-[oxybis(methylene)]-bis-2-propenoic acid
CH₂=C(COOH)CH₂-O-CH₂C(COOH)=CH₂
in a purity of at least 99% by hydrolysis of its esters in an aqueous medium and subsequent acidification of the resultant salt, which comprises carrying out the hydrolysis in water and in the presence of oxides or hydroxides of alkali or alkaline earth metals or ammonia.

2. A process as claimed in claim 1, wherein the hydrolysis is carried out in water and in the presence of sodium or potasium hydroxide.

3. A process as claimed in claim 1 or 2, wherein a polymerization inhibitor is used.

4. A process as claimed in any of claims 1 to 3, wherein the esters used of 2,2'-[oxybis(methylene)]bis-2-propenoic acid are one or more di(C₁-C₄-alkyl) esters.

5. A process as claimed in any of claims 1 to 4, wherein the crude product is recrystallized.

## Revendications

1. Procédé de préparation d'acide 2,2'-[oxy-bis-méthylène)]-bis-2-propénoïque
CH₂=C(COOH)CH₂-O-CH₂C(COOH)=CH₂
ayant un degré de pureté d'au moins 99%, par hydrolyse des esters correspondants en milieu aqueux, suivie d'acidification du sel formé, caractérisé en ce que l'hydrolyse est effectuée dans de l'eau et en présence d'oxydes ou d'hydroxydes de métaux alcalins ou alcalinoterreux ou d'ammoniac.

2. Procédé selon la revendication 1, caractérisé en ce que l'on effectue l'hydrolyse dans de l'eau en présence d'hydroxyde de sodium ou de potassium.

3. Procédé selon la revendication 1 ou 2, caractérisé en ce que l'on utilise simultanément un inhibiteur de polymérisation.

4. Procédé selon l'une quelconque des revendications 1 à 3, caractérisé en ce que l'on utilise, comme ester, un ou plusieurs di(esters d'alkyle en C₁-C₄) d'acide 2,2'-[oxy-bis-(méthylène)]-bis-2-propénoïque.

5. Procédé selon l'une quelconque des revendications 1 à 4, caractérisé en ce que l'on recristallise le produit brut obtenu.
